# EUROPEAN PATENT APPLICATION

(11) **EP 4 755 898 A1**
(43) Date of publication of application: **10.06.2026**
(21) Application number: 24849301.7
(22) Date of filing: 02.08.2024
(51) Int. Cl.: C07H 21/04

(54) **METHOD FOR PRODUCING OLIGONUCLEOTIDE**

(30) Priority: 02.08.2023 JP 2023126211
(71) Applicant: Nitto Denko Corporation, Ibaraki-shi, Osaka 567-8680 (JP)
(72) Inventor: YOSHIDA, Kei, Ibaraki-shi, Osaka 567-8680 (JP); MATSUOKA, Satoshi, Ibaraki-shi, Osaka 567-8680 (JP); IWAMOTO, Masafumi, Ibaraki-shi, Osaka 567-8680 (JP); IIDA, Tomoyoshi, Ibaraki-shi, Osaka 567-8680 (JP); MAETA, Eri, Ibaraki-shi, Osaka 567-8680 (JP); MATSUNAMI, Jun, Ibaraki-shi, Osaka 567-8680 (JP)
(74) Representative: Hoffmann Eitle
(86) International application number: PCT/JP2024/027749
(87) International publication number: WO 2025/028656

(57) **Abstract**

The purpose of the present invention is to provide a method for producing an oligonucleotide, wherein the amount of nucleoside phosphoramidite remaining in a reaction vessel is suppressed and the amount of used nucleoside phosphoramidite is reduced. The abovementioned problem is solved by, inter alia, a method for producing an oligonucleotide, the method comprising: (a) a step for detaching a protective group from a protected nucleoside where the protective group is bonded to a hydroxyl group, a thiol group, or an amino group at the 3' position or the 5' position; (b) a step for, in the presence of an activator, bonding a nucleoside phosphoramidite to the hydroxyl group, thiol group, or amino group at the 3' position or the 5' position of the nucleoside from which the protective group has been detached; (c) a step for sulfurizing or oxidizing the bond formed by step (b); and (d) a step for capping the hydroxyl group, thiol group, or amino group at the unbonded 3' position or 5' position of the nucleoside, wherein the capping of step (d) includes the operation of causing the nucleoside and a capping solution to come into contact for only a prescribed period of time [T] (minutes), the capping solution includes N-methyl imidazole at a blending rate of [N] (%) (the room temperature volume percentage (%) of unblended N-methyl imidazole with respect to the unblended room temperature total volume (100%) of reagents blended as capping solution components), and the product ([N] ×[T]) of [N] and [T] is 0.5 to 3.0.

## Description

### Technical Field

The present invention relates to a method for producing an oligonucleotide.

### Background Art

A phosphoramidite method is widely used in the chemical synthesis of nucleic acids such as DNA oligonucleotides and RNA oligonucleotides. In the phosphoramidite method, an oligonucleotide is typically synthesized by sequentially adding a nucleoside phosphoramidite to a nucleoside, a nucleotide, or an oligonucleotide in the presence of a suitable activating agent.

In the phosphoramidite method, nucleoside phosphoramidites are generally used in an amount of 1.5 to 10.0 times the theoretical amount. Since nucleoside phosphoramidites are expensive among synthetic raw materials, a significant reduction in production cost is expected when the amount of nucleoside phosphoramidites used can be reduced.

However, a method for obtaining an oligonucleotide with good purity while reducing the amount of nucleoside phosphoramidites used has not been known so far.

PTL 1 describes a method for synthesizing an oligonucleotide via a predetermined intermediate on a cleavable support having an azidomethyl moiety, addressing the issue that synthesis of an oligonucleotide often requires an excessive amount of an expensive reagent and is difficult to scale up.

PTL 2 describes a synthesis method using multi-base nucleotides, addressing the issue that a commercially available column synthesizer has a low material utilization rate, making it difficult to scale up nucleic acid synthesis at low cost.

PTL 3 describes a method for synthesizing an oligonucleotide using a highly lipophilic phosphoramidite, which is a type of alkoxyphosphoramidite compound usable as a capping reagent, addressing the issue that solid-phase synthesis is limited in scale-up and requires excessive amounts of a reaction reagent and a reaction raw material.

However, none of PTLs 1 to 3 focus on the conditions for the capping step, much less examine the relationship between the capping step and the residual amount of nucleoside phosphoramidites in a reaction vessel.

PTLs 4 to 7 and NPLs 1 and 2 describe various capping steps using a capping solution containing 1-methylimidazole, but these documents also do not examine at all the relationship between the capping step and the residual amount of nucleoside phosphoramidites in a reaction vessel.

PTL 8 describes that in the synthesis of a thiolated oligonucleotide, a process that excluded a capping step was superior to a comparative example in which a capping step was performed, but does not describe anything about the relationship between the capping step and the residual amount of nucleoside phosphoramidites in a reaction vessel.

### Citation List

### Patent Literature

PTL 1: WO2021/198883A
PTL 2: WO2019/158007A
PTL 3: JP7229539B
PTL 4: WO2022/132681A
PTL 5: CN115010769A
PTL 6: WO2002/014340A
PTL 7: WO2014/028739A
PTL 8: JP2019-518778A

### Non Patent Literature

NPL 1: BioTechniques 22(4): 752-756 (April 1997)
NPL 2: Molecules 2023, 28(7), 3265

### Summary of Invention

### Technical Problem

An object of the invention is to provide a method for producing an oligonucleotide, which suppresses the residual amount of nucleoside phosphoramidites in a reaction vessel and reduces the amount of nucleoside phosphoramidites used.

### Solution to Problem

The present inventors conducted intensive studies on a method for producing an oligonucleotide and found that by relaxing the conditions for the capping step, the residual amount of nucleoside phosphoramidites in a reaction vessel in a synthesis step can be suppressed, and the amount of nucleoside phosphoramidites used can be reduced. The present inventors conducted further studies based on this finding, and thus completed the invention.

That is, the invention relates to the following.
[1] A method for producing an oligonucleotide, the method including:
   (a) a step of detaching a protecting group from a protected nucleoside in which the protecting group is bonded to a hydroxy group, a thiol group, or an amino group at a 3'-position or a 5'-position;
   (b) a step of bonding a nucleoside phosphoramidite to the hydroxy group, the thiol group, or the amino group at the 3'-position or the 5'-position of the nucleoside, from which the protecting group has been detached, in the presence of an activating agent;
   (c) a step of sulfurizing or oxidizing a bond formed in the step (b); and
   (d) a step of capping an unbonded hydroxy group, thiol group, or amino group at the 3'-position or the 5'-position of the nucleoside, in which
      the capping in the step (d) includes an operation of bringing the nucleoside and a capping solution into contact with each other for only a predetermined time [T] (minutes),
      the capping solution contains N-methylimidazole at a blending ratio [N] (%) (which is a percentage (%) of a room temperature volume of N-methylimidazole before blending relative to a sum (100%) of room temperature volumes before blending of respective reagents to be blended as capping solution components), and
      a product of [N] and [T] ([N] × [T]) is 0.5 to 3.0.
[2] The method according to [1], in which the oligonucleotide has a sulfurized internucleoside bond.
[3] The method according to [1] or [2], in which [N] is 1.1 to 8.0 (%).
[4] The method according to any one of [1] to [3], in which [T] is 0.1 to 0.5 (minutes).
[5] The method according to any one of [1] to [4], in which the temperature of the solution in any of the steps (a) to (d) is 0 to 20°C.
[6] The method according to any one of [1] to [5], in which the amount of the activating agent used in the step (b) is 10.0 to 15.0 equivalents of the amount of nucleoside phosphoramidites used in the step (b).
[7] The method according to any one of [1] to [6], in which the amount of the activating agent used in the step (b) is 10.0 to 25.0 equivalents of the supported nucleoside in the step (b).
[8] The method according to any one of [1] to [7], in which the activating agent in the step (b) is selected from a group consisting of 4,5-dicyanoimidazole and 5-(ethylthio)-1H-tetrazole.

### Advantageous Effects of Invention

In a method for producing an oligonucleotide, by relaxing the conditions for a capping step, the residual amount of nucleoside phosphoramidites in a reaction vessel in a synthesis step can be suppressed, and an oligonucleotide can be obtained with good purity while reducing the amount of nucleoside phosphoramidites, which are expensive materials, used.

While not wishing to be bound by any particular theory, it is believed that the residual presence of nucleoside phosphoramidites in a reaction vessel results from the bonding of a nucleoside phosphoramidite in a coupling step to a hydroxy group formed by unintended detachment of a 2-cyanoethyl (CNET) protecting group at a phosphate moiety of an oligonucleotide, thereby preventing the nucleoside phosphoramidite from participating in a chain elongation reaction (a reaction with a 5'- or 3'-terminal hydroxy group of a nucleoside), whereas it is considered that by relaxing the conditions for a capping step, the bonding of a nucleoside phosphoramidite to a hydroxy group of an oligonucleotide formed by detachment of a CNET protecting group is suppressed, the amount of nucleoside phosphoramidites consumed in a reaction vessel is reduced, and thus a reduction in the amount of nucleoside phosphoramidites used is achieved. It should be noted that the residual presence of nucleoside phosphoramidites in a reaction vessel is not a phenomenon limited to a specific carrier or a specific reaction vessel.

### Description of Embodiments

Hereinafter, the invention will be described in detail.

Unless otherwise defined herein, all technical terms and scientific terms used herein have the same meanings as commonly understood by a person skilled in the art. All patents, applications, and other publications and information referenced herein are hereby incorporated by reference in their entirety. Further, when any inconsistency arises between the publications referenced herein and the description herein, the description herein shall prevail.

In one aspect, the invention relates to a method for producing an oligonucleotide.

In the invention, an oligonucleotide is produced using a so-called phosphoramidite method, in which a nucleotide is added by a condensation reaction of a nucleoside phosphoramidite with a nucleoside, a nucleotide, or an oligonucleotide in the presence of a suitable activating agent.

In the invention, the method for producing an oligonucleotide may include, for example:
(a) a step of detaching (deprotecting) a protecting group from a protected nucleoside in which the protecting group is bonded to a hydroxy group, a thiol group, or an amino group at a 3'-position or a 5'-position;
(b) a step of bonding (coupling) a nucleoside phosphoramidite to the hydroxy group, the thiol group, or the amino group at the 3'-position or the 5'-position of the nucleoside, from which the protecting group has been detached, in the presence of an activating agent;
(c) a step of sulfurizing or oxidizing a bond formed in the step (b); and
(d) a step of capping an unbonded hydroxy group, thiol group, or amino group at the 3'-position or the 5'-position of the nucleoside.

In the invention, the method for producing an oligonucleotide may include an additional step in addition to the above steps (a) to (d).

In one aspect of the invention, the method for producing an oligonucleotide includes:
(a) a step of detaching (deprotecting) a protecting group from a protected nucleoside in which the protecting group is bonded to a hydroxy group, a thiol group, or an amino group at a 3'-position or a 5'-position;
(b) a step of bonding (coupling) a nucleoside phosphoramidite to the hydroxy group, the thiol group, or the amino group at the 3'-position or the 5'-position of the nucleoside, from which the protecting group has been detached, in the presence of an activating agent;
(c) a step of sulfurizing or oxidizing a bond formed in the step (b); and
(d) a step of capping an unbonded hydroxy group, thiol group, or amino group at the 3'-position or the 5'-position of the nucleoside.

In the invention, the nucleoside refers to a compound in which a nucleoside base and a sugar are bonded together, and may be a naturally occurring nucleoside such as adenosine, thymidine, guanosine, cytidine, or uridine, or a modified nucleoside. Examples of the modified nucleoside include, but are not limited to, those in which a hydroxy group at the 3'-position or the 5'-position of a nucleoside has been substituted with a thiol group or an amino group. The nucleoside base may be a naturally occurring base such as adenine, guanine, cytosine, thymine, or uracil, or a modified nucleoside base. A sugar moiety of a nucleoside may be a naturally occurring deoxyribose or ribose or may have a D-configuration or an L-configuration.

In the invention, the nucleotide refers to a compound in which a nucleoside base, a sugar, and a phosphate are bonded together, and may be a naturally occurring nucleotide such as adenosine triphosphate, thymidine triphosphate, guanosine triphosphate, cytidine triphosphate, or uridine triphosphate, or a modified nucleotide. A nucleoside base moiety of a nucleotide may be a naturally occurring base such as adenine, guanine, cytosine, thymine, or uracil, or a modified nucleoside base. A sugar moiety of a nucleoside may be a naturally occurring deoxyribose or ribose or may have a D-configuration or an L-configuration. A phosphate moiety may be, for example, phosphorothioate, phosphorodithioate, methylphosphonate, or methyl phosphate.

In the invention, the oligonucleotide refers to a compound having a structure in which a nucleoside base, a sugar, and a phosphate are linked via a phosphodiester bond, and includes a naturally occurring oligonucleotide, for example, 2'-deoxyribonucleic acid (hereinafter referred to as "DNA") and ribonucleic acid (hereinafter referred to as "RNA"), as well as a nucleic acid containing a modified sugar moiety, a modified phosphate moiety, or a modified nucleobase. The modification of the sugar moiety include replacement of a ribose ring with a hexose, cyclopentyl, or cyclohexyl ring. Alternatively, a D-ribose ring of a naturally occurring nucleic acid may be replaced with an L-ribose ring, or a β-anomer of a naturally occurring nucleic acid may be replaced with an α-anomer. The oligonucleotide may also contain one or more abasic moieties. The modified phosphate moiety includes phosphorothioate, phosphorodithioate, methylphosphonate, and methyl phosphate. Such nucleic acid analogs are known to a person skilled in the art. An oligonucleotide including a mixture of two or more of the above can be produced, for example, from an oligonucleotide including a mixture of deoxyribo- and ribonucleosides, particularly a mixture of a deoxyribonucleoside and a 2'-O-substituted ribonucleoside such as a 2'-O-methyl or 2'-O-methoxyethyl ribonucleoside. Examples of the oligonucleotide including a mixture of nucleosides include a ribozyme.

In the invention, the nucleoside phosphoramidite refers to a nucleoside derivatized with an amidite. In the invention, in the nucleoside phosphoramidite, either a hydroxy group at the 3'-position or a hydroxy group at the 5'-position of a nucleoside is phosphoramidited, and a protecting group is bonded to the other hydroxy group.

The amidite formation can be carried out, for example, by allowing 2-cyanoethyl-N,N,N',N'-tetraisopropylphosphorodiamidite to react with a suitably protected nucleoside using 1H-tetrazole as an activating agent.

The nucleoside phosphoramidite may be a monomer or an oligomer such as a 2-mer to a 24-mer.

In the invention, the activating agent refers to an agent that activates a nucleoside phosphoramidite, and is used to react with a nucleoside, a nucleotide, or an oligonucleotide, and is also called an activator or a coupling agent. In the invention, an activating agent generally used in a phosphoramidite method can be used. Examples of the activating agent used in the invention include, but are not limited to, 4,5-dicyanoimidazole, 5-(ethylthio)-1H-tetrazole, 5-(benzylthio)-1H-tetrazole, and saccharin 1-methylimidazole, and 4,5-dicyanoimidazole and 5-(ethylthio)-1H-tetrazole are preferable.

The residual presence of nucleoside phosphoramidites in a reaction vessel is not a phenomenon limited to whether or not a protected nucleoside or a nucleoside described in the steps (a), (b), and (d) is supported on a carrier. In the invention, a protected nucleoside or a nucleoside described in the steps (a), (b), and (d) may or may not be supported on a carrier, but is preferably supported, for example, directly or indirectly on a carrier. In the invention, the "nucleoside directly supported on a carrier" refers to a nucleoside moiety (a compound moiety in which a nucleoside base and a sugar are bonded) of a compound in which a nucleoside or a nucleotide is bonded to a reactive site of a carrier, and the "nucleoside indirectly supported on a carrier" refers to a nucleoside moiety (a compound moiety in which a nucleoside base and a sugar are bonded) in which a nucleotide is bonded to a reactive site of a carrier via a compound such as a polynucleotide. The residual presence of nucleoside phosphoramidites in a reaction vessel is not a phenomenon limited to a specific carrier or a specific reaction vessel, and the carrier and the reaction vessel used in the invention are also not particularly limited in type or shape. The residual presence of nucleoside phosphoramidites in a reaction vessel is not a phenomenon limited to either of a solid-phase synthesis method and a liquid-phase synthesis method, and the invention can be applied to both a solid-phase synthesis method and a liquid-phase synthesis method, but, for example, application to a solid-phase synthesis method is preferable. In the invention, the "carrier" is not particularly limited in type or shape as long as it can directly or indirectly support a nucleoside, but, for example, a carrier for solid-phase synthesis or a carrier for liquid-phase synthesis is preferable. In the invention, as the carrier for solid-phase synthesis, for example, a polymer-based carrier, a glass-based carrier, a silicon-based carrier, or the like is preferable, a polymer-based carrier or a glass-based carrier is more preferable. In the case of a polymer-based carrier, the type is not limited, but a polystyrene-based carrier is more preferable. As the shape of the carrier for solid-phase synthesis, any shape such as a particle, a rod, or a plate can be selected, and they may have a porous structure, for example, a porous particle is preferable. The carrier for solid-phase synthesis is preferably a polymer-based or glass-based porous particle, and in the case of a polymer-based porous particle, the type is not limited, but a polystyrene-based porous particle is more preferable. In the invention, as the carrier for liquid-phase synthesis, a soluble carrier, a pseudo-solid-phase carrier, or a pseudo-solid-phase protecting group is preferable. In the invention, which site of a nucleoside or a nucleotide binds to a reactive site of a carrier, that is, the binding site, is not particularly limited. The binding site can be appropriately selected according to the type of carrier for solid-phase synthesis or carrier for liquid-phase synthesis. In the invention, the type and shape of the "reaction vessel" are not particularly limited as long as the carrier and the reaction components can be held for a certain period of time. A vessel (such as a column) containing a material selected from the group consisting of a glass, stainless steel, polyether ether ketone, and the like, and a combination thereof can be suitably used.

In the method for producing an oligonucleotide according to the invention, the conditions for the capping step are relaxed. The relaxation of the conditions for the capping step can be indicated by the product ([N] × [T]) of the predetermined contact time [T] (minutes) and the blending ratio [N] (%) of N-methylimidazole contained in the capping solution (which is a percentage (%) of a room temperature volume of N-methylimidazole before blending relative to a sum (100%) of room temperature volumes before blending of respective reagents to be blended as capping solution components) in an operation of bringing the nucleoside and the capping solution into contact with each other as an index. That the conditions for the capping step are relaxed refers to, for example, the product of [N] and [T] ([N] × [T]) being about 0.5 to 3.0.

In one aspect of the invention, the method for producing an oligonucleotide includes:
(a) a step of detaching a protecting group from a protected nucleoside in which the protecting group is bonded to a hydroxy group, a thiol group, or an amino group at a 3'-position or a 5'-position;
(b) a step of bonding a nucleoside phosphoramidite to the hydroxy group, the thiol group, or the amino group at the 3'-position or the 5'-position of the nucleoside, from which the protecting group has been detached, in the presence of an activating agent;
(c) a step of sulfurizing or oxidizing a bond formed in the step (b); and
(d) a step of capping an unbonded hydroxy group, thiol group, or amino group at the 3'-position or the 5'-position of the nucleoside, and
   in the method, the capping in the step (d) includes an operation of bringing the nucleoside and a capping solution into contact with each other for only a predetermined time [T] (minutes),
   the capping solution contains N-methylimidazole at a blending ratio [N] (%) (which is a percentage (%) of a room temperature volume of N-methylimidazole before blending relative to a sum (100%) of room temperature volumes before blending of respective reagents to be blended as capping solution components), and
   a product of [N] and [T] ([N] × [T]) is 0.5 to 3.0.

The contact between the nucleoside and the capping solution can be carried out according to a conventional method. In a typical contact method, multiple types of liquids such as a cap A liquid and a cap B liquid are mixed immediately before in a flow channel and introduced into a reaction vessel (such as a column), but these liquids may also be mixed in the reaction vessel. The contact time in the invention refers to a time required for the capping solution to pass through the inside of the reaction vessel after mixing.

In one aspect of the invention, the product of [N] and [T] ([N] × [T]) is not particularly limited as long as the conditions for the capping step are relaxed, but is preferably, for example, 0.5 to 3.0. Specifically, the product is 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0, and may be in a range between any two of these numerical values.

In one aspect of the invention, the blending ratio [N] (%) of N-methylimidazole contained in the capping solution is not particularly limited, but may be, for example, 1.0 to 30 (%), and is preferably 1.1 to 8.0 (%). Specifically, the blending ratio [N] is 1.1 (%), 1.4 (%), 1.5 (%), 2.0 (%), 2.5 (%), 3.0 (%), 3.3 (%), 3.5 (%), 4.0 (%), 4.5 (%), 5.0 (%), 5.5 (%), 6.0 (%), 6.5 (%), 7.0 (%), 7.5 (%), or 8.0 (%), and may be in a range between any two of these numerical values.

In one aspect of the invention, [T] is not particularly limited, but may be, for example, 0.05 to 3 (minutes), and is preferably 0.1 to 0.5 (minutes). Specifically, [T] is 0.1 (minutes), 0.15 (minutes), 0.2 (minutes), 0.25 (minutes), 0.3 (minutes), 0.35 (minutes), 0.4 (minutes), 0.45 (minutes), or 0.5 (minutes), and may be in a range between any two of these numerical values.

In one aspect of the invention, the type of internucleoside bond contained in the produced oligonucleotide is not particularly limited, but for example, the produced oligonucleotide preferably has a sulfurized internucleoside bond. For example, it is preferable that at least one of the bonds formed in the step (b) is sulfurized, and specifically, 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, or 30 or more of the bonds may be sulfurized. More preferably, 6 or more of the bonds formed in the step (b) may be sulfurized. More preferably, 12 or more of the bonds formed in the step (b) may be sulfurized. Still more preferably, 17 or more of the bonds formed in the step (b) may be sulfurized. Still more preferably, 18 or more of the bonds formed in the step (b) may be sulfurized.

In one aspect of the invention, the type of internucleoside bond contained in the produced oligonucleotide is not particularly limited. For example, the produced oligonucleotide may have an oxidized internucleoside bond, but preferably has a sulfurized internucleoside bond. For example, it is preferable that at least one site of the bonds formed in the step (b) is sulfurized, and specifically, 5% or more, 10% or more, 15% or more, 20% or more, 25% or more, 30% or more, 35% or more, 40% or more, 45% or more, 50% or more, 55% or more, 60% or more, 65% or more, 70% or more, 74% or more, 75% or more, 78% or more, 80% or more, 85% or more, 90% or more, 95% or more, or 100% of the sites may be sulfurized. More preferably, 25% or more of the sites of the bonds formed in the step (b) may be sulfurized. More preferably, 50% or more of the sites of the bonds formed in the step (b) may be sulfurized. Still more preferably, 74% or more of the sites of the bonds formed in the step (b) may be sulfurized. Still more preferably, 78% or more of the sites of the bonds formed in the step (b) may be sulfurized. Still more preferably, 100% of the bonds formed in the step (b) may be sulfurized.

Independently of the type of internucleoside bond contained in the above-mentioned oligonucleotide, although the structure at the 3'-terminal of the oligonucleotide is not limited, when the oligonucleotide has a phosphate structure at the 3'-terminal, the phosphate structure may or may not be sulfurized, but is preferably sulfurized.

In one aspect of the invention, the amount of the nucleoside phosphoramidites used in the step (b) is not particularly limited, but is preferably, for example, 1.0 to 2.0 equivalents, more preferably about 1.1 to about 1.7 equivalents, and still more preferably about 1.2 equivalents of the supported nucleoside. Specifically, the amount is 1.0 equivalents, 1.1 equivalents, 1.12 equivalents, 1.13 equivalents, 1.14 equivalents, 1.15 equivalents, 1.16 equivalents, 1.17 equivalents, 1.18 equivalents, 1.19 equivalents, 1.2 equivalents, 1.3 equivalents, 1.4 equivalents, 1.5 equivalents, 1.6 equivalents, 1.7 equivalents, 1.8 equivalents, 1.9 equivalents, or 2.0 equivalents, or may be within a range between any two of these numerical values.

In one aspect of the invention, the amount of the activating agent used in the step (b) is not particularly limited, but is preferably, for example, 2.0 to 15.0 equivalents, and more preferably 10.0 to 15.0 equivalents of the amount of nucleoside phosphoramidites used in the step. Specifically, the amount is 2.0 equivalents, 2.5 equivalents, 3.0 equivalents, 3.5 equivalents, 4.0 equivalents, 4.5 equivalents, 5.0 equivalents, 5.5 equivalents, 6.0 equivalents, 6.5 equivalents, 7.0 equivalents, 7.5 equivalents, 8.0 equivalents, 8.5 equivalents, 9.0 equivalents, 9.5 equivalents, 10.0 equivalents, 10.5 equivalents, 11.0 equivalents, 11.5 equivalents, 12.0 equivalents, 12.5 equivalents, 13.0 equivalents, 13.5 equivalents, 14.0 equivalents, 14.5 equivalents, or 15.0 equivalents, or may be within a range between any two of these numerical values.

In one aspect of the invention, the amount of the activating agent used in the step (b) is not particularly limited, but is preferably, for example, 10.0 to 25.0 equivalents of the supported nucleoside. Specifically, the amount is 10.0 equivalents, 10.5 equivalents, 11.0 equivalents, 11.5 equivalents, 12.0 equivalents, 12.5 equivalents, 13.0 equivalents, 13.5 equivalents, 14.0 equivalents, 14.5 equivalents, 15.0 equivalents, 15.5 equivalents, 16.0 equivalents, 16.5 equivalents, 17.0 equivalents, 17.5 equivalents, 18.0 equivalents, 18.5 equivalents, 19.0 equivalents, 19.5 equivalents, 20.0 equivalents, 20.5 equivalents, 21.0 equivalents, 21.5 equivalents, 22.0 equivalents, 22.5 equivalents, 23.0 equivalents, 23.5 equivalents, 24.0 equivalents, 24.5 equivalents, or 25.0 equivalents, or may be within a range between any two of these numerical values.

In one aspect of the invention, the temperature of the solution in any of the steps (a) to (d) is not particularly limited, but is preferably 0 to 30°C, more preferably 0 to 20°C, and still more preferably 5 to 20°C. Specifically, the temperature is 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 22.5°C, 23°C, 24°C, 25°C, 26°C, 27°C, 28°C, 29°C, or 30°C, or may be within a range between any two of these numerical values.

Examples of a method for controlling the temperature of the solution include a method in which a solution previously controlled to a desired temperature is supplied in each step, a method in which a solution is supplied in each step and then controlled to a desired temperature by cooling or the like of the reaction vessel, and a method in which a pipe is provided inside the reaction vessel and a refrigerant or the like is allowed to pass through the pipe to control the temperature to a desired level. Further, in the invention, the temperature when the solution previously controlled to a desired temperature is supplied in each step is referred to as a supply liquid temperature.

In one aspect of the invention, a component further contained in the capping solution is not particularly limited, but is preferably, for example, a base, an acetylating agent, or a solvent.

In one aspect of the invention, the type of base that can be included in the capping solution components is not particularly limited, but is preferably, for example, 2,6-lutidine or pyridine. The blending ratio (%) of the base (which is a percentage (%) of a room temperature volume of the base before blending relative to the sum (100%) of room temperature volumes before blending of respective reagents to be blended as capping solution components) is not particularly limited, but is preferably, for example, 3 to 30%, and more preferably 5 to 25%. Specifically, the blending ratio is 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%, or may be within a range between any two of these numerical values.

In one aspect of the invention, the type of acetylating agent that can be included in the capping solution components is not particularly limited, but is preferably, for example, acetic anhydride. The blending ratio (%) of the acetylating agent (which is a percentage (%) of a room temperature volume of the acetylating agent before blending relative to the sum (100%) of room temperature volumes before blending of respective reagents to be blended as capping solution components) is not particularly limited, but is preferably, for example, 2 to 20%, more preferably 2.5 to 15%, and more preferably 5 to 15%. Specifically, the blending ratio is 2%, 2.5%, 3%, 4%, 5%, 6%, 7%, 8%, 9%, 10%, 11%, 12%, 13%, 14%, 15%, 16%, 17%, 18%, 19%, or 20%, or may be within a range between any two of these numerical values.

In one aspect of the invention, the type of solvent that can be included in the capping solution components is not particularly limited, but is preferably, for example, an organic solvent. Examples of the organic solvent include acetonitrile and tetrahydrofuran.

In this description, the residual amount of nucleoside phosphoramidites in a reaction vessel refers to the amount of nucleoside phosphoramidites remaining in a reaction vessel in the step of synthesizing an oligonucleotide, for example, due to the theory explained above, or the like. The residual amount of nucleoside phosphoramidites in a reaction vessel can be measured, for example, by recovering the waste liquid in the oxidation step or the sulfurization step in each synthesis cycle, and optionally a washing liquid after a coupling reaction in the synthesis cycle, and analyzing the amount of a DMTr (4,4'-dimethoxytriphenylmethyl group) protecting group of nucleoside phosphoramidites by HPLC.

### Examples

The invention will be described in more detail with reference to the following examples, but these illustrate particular specific examples of the invention, and the invention is not limited thereto.

### <Materials and Methods>

The oligonucleotides used in Examples and Comparative Examples are as follows.

**[Table 1]**

| SEQ ID NO | Sequence and modification |
|---|---|
| 1 | 5'-TCGACGTATTGACGTATTGACGTA-3', all linkage portions: PS (also 3'-terminal P: PS) |
| 2 | 5'-TCGACGTATTGACGTATTGACGTA-3', all linkage portions: PO (also 3'-terminal P: PO) |
| 3 | 5'-CGTACGTACGTACGTACGTACGTA-3', all linkage portions: PS (also 3'-terminal P: PS) |
| 4 | 5'-TATACCGATTAAGCGAAGTTT-3', all linkage portions: PS (also 3'-terminal P: PS) |
| 5 | 5'-TTTGAAGCGAATTAGCCATATCGTA-3', all linkage portions: PS (also 3'-terminal P: PS) |
| 6 | 5'-GGUUUAACGUACGUACGUACGUA-3', all 2'-OMe modification, all linkage portions: PS (also 3'-terminal P: PS) |
| 7 | 5'-TCGACGTATTGACGTATTGACGTA-3', 1st to 17th linkage portions from 3'-terminal: PS, 18th to 23rd linkage portions from 3'-terminal: PO (3'-terminal P: PS) |
| 8 | 5'-TCGACGTATTGACGTATTGACGTA-3', 3rd to 20th linkage portions from 3'-terminal: PS, 1st, 2nd, and 21st to 23rd linkage portions from 3'-terminal: PO (3'-terminal P: PO) |

| | |
|---|---|
| linkage portion: internucleoside bond | |

### 1. SEQ ID NO: 1

### (1) Synthesis of DNA Oligonucleotide (SEQ ID NO: 1)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Tables 2 to 7, and a coupling reaction (condensation time: 5 minutes) was performed under the conditions shown in Tables 2 to 7. The activating agent was all dissolved in acetonitrile and prepared to 0.7 M. The other synthetic reagents used were 3% DCA in toluene as a deprotecting agent, 0.2 M xanthan hydride in pyridine prepared as a sulfurizing agent, a mixed solution of lutidine or pyridine, N-methylimidazole, and acetic anhydride in acetonitrile as a capping agent, and TBA in acetonitrile (in 8:2 ratio) as an amine wash reaction solution. A 24-mer DNA oligonucleotide (5'-TCGACGTATTGACGTATTGACGTA-3' in which the phosphite esters (internucleoside bonds) and the 3'-terminal phosphate were all sulfurized: SEQ ID NO: 1) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent in the sulfurization step and discharged together with the waste liquid. Therefore, all waste liquid in the sulfurization step in the synthesis of the 24-mer DNA, that is, each of the 24 synthesis cycles in total was recovered. After the coupling reaction in the 24th synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 2. SEQ ID NO: 2

### (1) Synthesis of DNA Oligonucleotide (SEQ ID NO: 2)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Table 6, and a coupling reaction (condensation time: 10 minutes) was performed under the conditions shown in Table 6. The activating agent was all dissolved in acetonitrile and prepared to 0.7 M. The other synthetic reagents used were 3% DCA in toluene as a deprotecting agent, an iodine solution (0.05 mol/L: the solvent was pyridine : water in (9:1) ratio) as an oxidizing agent, a mixed solution of lutidine, N-methylimidazole, and acetic anhydride in acetonitrile as a capping agent, and TBA in acetonitrile (in 8:2 ratio) as an amine wash reaction solution. A 24-mer DNA oligonucleotide (5'-TCGACGTATTGACGTATTGACGTA-3' in which the phosphite esters (internucleoside bonds) and the 3'-terminal phosphate were all oxidized: SEQ ID NO: 2) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the oxidizing agent in the oxidation step and discharged together with the waste liquid. Therefore, all waste liquid in the oxidation step in the synthesis of the 24-mer DNA, that is, each of the 24 synthesis cycles in total was recovered. After the coupling reaction in the 24th synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 3. SEQ ID NO: 3

### (1) Synthesis of DNA Oligonucleotide (SEQ ID NO: 3)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Table 7, and a coupling reaction (condensation time: 5 minutes) was performed under the conditions shown in Table 7. The activating agent was all dissolved in acetonitrile and prepared to 0.7 M. The other synthetic reagents used were 3% DCA in toluene as a deprotecting agent, 0.2 M xanthan hydride in pyridine prepared as a sulfurizing agent, a mixed solution of lutidine, N-methylimidazole, and acetic anhydride in acetonitrile as a capping agent, and TBA in acetonitrile (in 8:2 ratio) as an amine wash reaction solution. A 24-mer DNA oligonucleotide (5'-CGTACGTACGTACGTACGTACGTA-3' in which the phosphite esters (internucleoside bonds) and the 3'-terminal phosphate were all sulfurized: SEQ ID NO: 3) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent in the sulfurization step and discharged together with the waste liquid. Therefore, all waste liquid in the sulfurization step in the synthesis of the 24-mer DNA, that is, each of the 24 synthesis cycles in total was recovered. After the coupling reaction in the 24th synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 4. SEQ ID NO: 4

### (1) Synthesis of DNA Oligonucleotide (SEQ ID NO: 4)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Table 7, and a coupling reaction (condensation time: 5 minutes) was performed under the conditions shown in Table 7. The activating agent was all dissolved in acetonitrile and prepared to 0.7 M. The other synthetic reagents used were 3% DCA in toluene as a deprotecting agent, 0.2 M xanthan hydride in pyridine prepared as a sulfurizing agent, a mixed solution of lutidine, N-methylimidazole, and acetic anhydride in acetonitrile as a capping agent, and TBA in acetonitrile (in 8:2 ratio) as an amine wash reaction solution. A 21-mer DNA oligonucleotide (5'-TATACCGATTAAGCGAAGTTT-3' in which the phosphite esters (internucleoside bonds) and the 3'-terminal phosphate were all sulfurized: SEQ ID NO: 4) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent in the sulfurization step and discharged together with the waste liquid. Therefore, all waste liquid in the sulfurization step in the synthesis of the 21-mer DNA, that is, each of the 21 synthesis cycles in total was recovered. After the coupling reaction in the 21st synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 5. SEQ ID NO: 5

### (1) Synthesis of DNA Oligonucleotide (SEQ ID NO: 5)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Table 7, and a coupling reaction (condensation time: 5 minutes) was performed under the conditions shown in Table 7. The activating agent was all dissolved in acetonitrile and prepared to 0.7 M. The other synthetic reagents used were 3% DCA in toluene as a deprotecting agent, 0.2 M xanthan hydride in pyridine prepared as a sulfurizing agent, a mixed solution of lutidine, N-methylimidazole, and acetic anhydride in acetonitrile as a capping agent, and TBA in acetonitrile (in 8:2 ratio) as an amine wash reaction solution. A 25-mer DNA oligonucleotide (5'-TTTGAAGCGAATTAGCCATATCGTA-3' in which the phosphite esters (internucleoside bonds) and the 3'-terminal phosphate were all sulfurized: SEQ ID NO: 5) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent in the sulfurization step and discharged together with the waste liquid. Therefore, all waste liquid in the sulfurization step in the synthesis of the 25-mer DNA, that is, each of the 25 synthesis cycles in total was recovered. After the coupling reaction in the 25th synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 6. SEQ ID NO: 6

### (1) Synthesis of RNA Oligonucleotide (SEQ ID NO: 6)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 5-(ethylthio)-1H-tetrazole (ETT) as an activating agent were charged in the amounts shown in Table 7, and a coupling reaction (condensation time: 10 minutes) was performed under the conditions shown in Table 7. The activating agent was all dissolved in acetonitrile and prepared to 0.6 M. The other synthetic reagents used were 3% DCA in toluene as a deprotecting agent, 0.2 M xanthan hydride in pyridine prepared as a sulfurizing agent, a mixed solution of lutidine, N-methylimidazole, and acetic anhydride in acetonitrile as a capping agent, and TBA in acetonitrile (in 8:2 ratio) as an amine wash reaction solution. A 23-mer 2'-OMe-RNA oligonucleotide (5'-GGUUUAACGUACGUACGUACGUA-3' in which the phosphite esters (internucleoside bonds) and the 3'-terminal phosphate were all sulfurized: SEQ ID NO: 6) was synthesized, and the terminal DMTr protecting group was removed. The porous resin beads with the RNA oligonucleotide bonded thereto were dried. Thereafter, the porous resin beads were immersed in aqueous ammonia, the RNA oligonucleotide was cleaved from the porous resin beads, the base amino group was deprotected, and a filtrate in which the 2'-OMe-RNA oligonucleotide was dissolved was obtained.

### (2) Measurement of Purity of Synthesized RNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent in the sulfurization step and discharged together with the waste liquid. Therefore, all waste liquid in the sulfurization step in the synthesis of the 23-mer 2'-OMe-RNA, that is, each of the 23 synthesis cycles in total was recovered. After the coupling reaction in the 23rd synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 7. SEQ ID NO: 7

### (1) Synthesis of DNA Oligonucleotide (SEQ ID NO: 7)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Table 6, and a coupling reaction (condensation time: 5 minutes for cycles including a sulfurization step, 10 minutes for cycles including an oxidation step) was performed under the conditions shown in Table 6. The activating agent was all dissolved in acetonitrile and prepared to 0.7 M. The other synthetic reagents used were 3% DCA in toluene as a deprotecting agent, 0.2 M xanthan hydride in pyridine prepared as a sulfurizing agent, an iodine solution (0.05 mol/L: the solvent was pyridine : water in (9:1) ratio) as an oxidizing agent, a mixed solution of lutidine, N-methylimidazole, and acetic anhydride in acetonitrile as a capping agent, and TBA in acetonitrile (in 8:2 ratio) as an amine wash reaction solution. A 24-mer DNA oligonucleotide (5'-TCGACGTATTGACGTATTGACGTA-3' in which the 1st to 17th phosphite esters (internucleoside bonds) from the 3'-terminal and the 3'-terminal phosphate were sulfurized, and the 18th to 23rd phosphite esters from the 3'-terminal were oxidized: SEQ ID NO: 7) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent or the oxidizing agent in the sulfurization step or the oxidation step and discharged together with the waste liquid. Therefore, all waste liquid in the sulfurization step or the oxidation step in the synthesis of the 24-mer DNA, that is, each of the 24 synthesis cycles in total was recovered. After the coupling reaction in the 24th synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### 8. SEQ ID NO: 8

### (1) Synthesis of DNA Oligonucleotide (SEQ ID NO: 8)

Porous resin beads (NittoPhase (registered trademark) HL UnyLinker 350) were placed in a synthesis column (volume: 12.6 ml) so that the synthesis scale (the total reactive sites of the beads) was 480 µmol, and the synthesis column was set in an AKTA oligopilot plus 100 synthesizer (manufactured by Cytiva), nucleoside phosphoramidites and 4,5-dicyanoimidazole (DCI) as an activating agent were charged in the amounts shown in Table 6, and a coupling reaction (condensation time: 5 minutes for cycles including a sulfurization step, 10 minutes for cycles including an oxidation step) was performed under the conditions shown in Table 6. The activating agent was all dissolved in acetonitrile and prepared to 0.7 M. The other synthetic reagents used were 3% DCA in toluene as a deprotecting agent, 0.2 M xanthan hydride in pyridine prepared as a sulfurizing agent, an iodine solution (0.05 mol/L: the solvent was pyridine : water in (9:1) ratio) as an oxidizing agent, a mixed solution of lutidine, N-methylimidazole, and acetic anhydride in acetonitrile as a capping agent, and TBA in acetonitrile (in 8:2 ratio) as an amine wash reaction solution. A 24-mer DNA oligonucleotide (5'-TCGACGTATTGACGTATTGACGTA-3' in which the 3rd to 20th phosphite esters (internucleoside bonds) from the 3'-terminal were sulfurized, and the 1st, 2nd, and 21st to 23rd phosphite esters (internucleoside bonds) from the 3'-terminal and the 3'-terminal phosphate were oxidized: SEQ ID NO: 8) was synthesized, and the terminal DMTr protecting group was removed. The porous resin beads with the DNA oligonucleotide bonded thereto were dried. Thereafter, the porous resin beads were immersed in aqueous ammonia, the DNA oligonucleotide was cleaved from the porous resin beads, the base amino group was deprotected, and a filtrate in which the DNA oligonucleotide was dissolved was obtained.

### (2) Measurement of Purity of Synthesized DNA Oligonucleotide

An oligonucleotide sample filtrate was prepared to OD 5 and subjected to measurement by high performance liquid chromatography (HPLC) under the following conditions. The peak area (%) of the main component was defined as the synthesis purity (Full-length: area%), with the sum of peak areas from detection of the main component to about 10 minutes being taken as 100%.
Column: manufactured by Waters Corporation, ACQUITY UPLC Oligonucleotide BEH C18 Column, 130 Å, 1.7 µm, 2.1 mm × 100 mm
UV detection: 260 nm
Mobile phase A: 400 mM HFIP/15 mM TEA aqueous solution
Mobile phase B: methanol
Column temperature: 60°C

### (3) Measurement of Amount of Nucleoside Phosphoramidites Remaining in Column

A nucleoside phosphoramidite bonded to a phosphate moiety of an oligonucleotide was detached with the sulfurizing agent or the oxidizing agent in the sulfurization step or the oxidation step and discharged together with the waste liquid. Therefore, all waste liquid in the sulfurization step or the oxidation step in the synthesis of the 24-mer DNA, that is, each of the 24 synthesis cycles in total was recovered. After the coupling reaction in the 24th synthesis cycle, the inside of the column was washed with 5 column volumes (63 ml) of acetonitrile for thorough washing, and this was also recovered as the waste liquid. The amount of nucleoside phosphoramidites was estimated from the absolute amount of the DMTr protecting group of the nucleoside phosphoramidites. The nucleoside phosphoramidites were hydrolyzed to obtain the DMTr protecting group, which was appropriately diluted with an acetonitrile solution of 0.1 M para-toluenesulfonic acid monohydrate (pTSA), and then, the absolute amount of the DMTr protecting group was measured by HPLC under the following conditions.
Column: manufactured by Waters Corporation, Atlantis T3, 130 Å, 3.0 µm, 2.1 mm × 150 mm
MS detection: ESI-Posi. m/z 303
Mobile phase A: 0.1% formic acid aqueous solution
Mobile phase B: acetonitrile
Column temperature: 40°C

The residual rate of nucleoside phosphoramidites in the column was calculated by dividing the absolute amount of the DMTr protecting group in the waste liquid (corresponding to the amount of bonded nucleoside phosphoramidites) by the amount of nucleoside phosphoramidites charged into the reaction (synthesis scale × nucleoside phosphoramidite equivalent). This waste liquid refers to the waste liquid discharged in the sulfurization step or the oxidation step.

### <Results and Discussion>

### 1. Evaluation of Suitable Range of [N] × [T]

**[Table 2]**

| | Blending ratio of NMI in capping solution [N] (%) | Capping time [T] (min) | [N] × [T] | Evaluation results | | | | |
|---|---|---|---|---|---|---|---|---|
| | | | | Residual rate in column | | Purity of final product | | Comparative target |
| | | | | Residual rate (%) | Percentage relative to comparative example (%) | Purity (%) | Increase point relative to comparative example | |
| Comparative Example 1 | 10.0 | 0.50 | 5.0 | 19.9 | 100 | 80.2 | 0 | - |
| Example 1 | 8.0 | 0.38 | 3.0 | 18.6 | 93 | 82.1 | 1.9 | relative to Comparative Example 1 |
| Example 2 | 4.0 | 0.50 | 2.0 | 14.7 | 74 | 83.9 | 3.7 | relative to Comparative Example 1 |
| Example 3 | 4.5 | 0.38 | 1.7 | 12.8 | 64 | 84.0 | 3.8 | relative to Comparative Example 1 |
| Example 4 | 4.0 | 0.38 | 1.5 | 16.3 | 82 | 84.4 | 4.2 | relative to Comparative Example 1 |
| Example 5 | 3.3 | 0.38 | 1.3 | 14.3 | 72 | 84.6 | 4.4 | relative to Comparative Example 1 |
| Example 6 | 2.0 | 0.38 | 0.8 | 13.5 | 68 | 82.6 | 2.4 | relative to Comparative Example 1 |
| Example 7 | 1.4 | 0.38 | 0.5 | 14.2 | 71 | 82.8 | 2.6 | relative to Comparative Example 1 |
| Comparative Example 2 | 1.0 | 0.38 | 0.4 | 23.2 | 117 | 77.7 | -2.5 | relative to Comparative Example 1 |
| Comparative Example 3 | Capping was not performed. | | | 20.2 | 102 | 78.8 | -1.4 | relative to Comparative Example 1 |

| | | | | | | | | |
|---|---|---|---|---|---|---|---|---|
| Oligonucleotide: SEQ ID NO: 1, Supply liquid temperature during whole process: 22.5°C, Charged amount of nucleoside phosphoramidites: 1.2 equivalents, Activating agent: DCI (4,5-dicyanoimidazole): 4.2 equivalents, Charged amount ratio of (activating agent/nucleoside phosphoramidites): 3.5 times, Blending ratio of capping solution component (base): 15% lutidine, Blending ratio of capping solution component (acetylating agent): 10% acetic anhydride NMI: N-methylimidazole | | | | | | | | |

As the evaluation results of Examples 1 to 7 and Comparative Examples 1 to 3, the residual rate (%) in the column and the purity (%) of the final product are shown in Table 2. As for the residual rate in the column of each of Examples 1 to 7 and Comparative Examples 2 and 3, a percentage (%) when the residual rate in the column of Comparative Example 1, in which the comparative target capping conditions ([N] × [T] = 5.0) were used, was taken as 100% was also calculated and shown together. When these values are less than 100%, it indicates that the residual rate in the column is suppressed as compared with that of Comparative Example 1. As for the purity of the final product of each of Examples 1 to 7 and Comparative Examples 2 and 3, a difference (point) from the purity of the final product of Comparative Example 1 was also calculated and shown together. When these values are positive values, it indicates that the purity of the final product is improved as compared with that of Comparative Example 1.

In Examples 1 to 7 in which the value of [N] × [T] was in the range of 0.5 to 3.0, the percentage of the residual rate in the column relative to Comparative Example 1 was less than 100%, indicating that the residual rate in the column was suppressed, and further, the increase point of the purity of the final product relative to Comparative Example 1 was a positive value, indicating that the purity of the final product was improved.

On the other hand, in both Comparative Example 2 in which the value of [N] × [T] was 0.4, and Comparative Example 3 in which capping was not performed, the percentage of the residual rate in the column relative to Comparative Example 1 was 100% or more, indicating that the residual rate in the column was not suppressed, and the increase point of the purity of the final product relative to Comparative Example 1 was a negative value, indicating that the purity of the final product was not improved.

From the above results, it was found that in the production of an oligonucleotide, by performing capping under relaxed capping conditions in which the value of [N] × [T] is 0.5 to 3.0, rather than not performing capping at all, the residual rate of nucleoside phosphoramidites in the column can be suppressed, and moreover, the purity of the final synthesized oligonucleotide product can be improved.

### 2. Evaluation of Charged Amount of Nucleoside Phosphoramidites

**[Table 3]**

| | Charged amount of amidites (equivale nts) | Activating agent | | Charged amount ratio of (activating agent/amid ites) (times) | Blendi ng ratio of NMI in cappi ng soluti on [N] (%) | Cappi ng time [T] (min) | [N ] × [T ] | Evaluation results | | | | |
|---|---|---|---|---|---|---|---|---|---|---|---|---|
| | | Ty pe | Charged amount (equivale nts) | | | | | Residual rate in column | | Purity of final product | | Compara tive target |
| | | | | | | | | Resid ual rate (%) | Percent age relative to compara tive example (%) | Puri ty (%) | Increase point relative to compara tive example | |
| Compara tive Example 1 | 1.2 | DC I | 4.2 | 3.5 | 10.0 | 0.50 | 5. 0 | 19.9 | 100 | 80. 2 | 0 | - |
| Example 5 | 1.2 | DC I | 4.2 | 3.5 | 3.3 | 0.38 | 1. 3 | 14.3 | 72 | 84. 6 | 4.4 | relative to Compara tive Example 1 |
| Compara tive Example 13 | 1.6 | DC I | 5.6 | 3.5 | 10.0 | 0.50 | 5. 0 | 28.5 | 100 | 84. 8 | 0 | - |
| Example 20 | 1.6 | DC I | 5.6 | 3.5 | 3.3 | 0.38 | 1. 3 | 22.4 | 79 | 85. 5 | 0.7 | relative to Compara tive Example 13 |
| Compara tive Example 4 | 1.7 | DC I | 6.0 | 3.5 | 10.0 | 0.50 | 5. 0 | 33.3 | 100 | 85. 8 | 0 | - |
| Example 8 | 1.7 | DC I | 6.0 | 3.5 | 3.3 | 0.38 | 1. 3 | 25.6 | 77 | 85. 9 | 0.1 | relative to Compara tive Example 4 |

| | | | | | | | | | | | | |
|---|---|---|---|---|---|---|---|---|---|---|---|---|
| Oligonucleotide: SEQ ID NO: 1, Supply liquid temperature during whole process: 22.5°C, Activating agent: DCI (4,5-dicyanoimidazole), Amidite: nucleoside phosphoramidite Blending ratio of capping solution component (base): 15% lutidine, Blending ratio of capping solution component (acetylating agent): 10% acetic anhydride NMI: N-methylimidazole | | | | | | | | | | | | |

As the evaluation results of Examples 5, 20, and 8 and Comparative Examples 1, 13, and 4, the residual rate (%) in the column and the purity (%) of the final product are shown in Table 3. As for the residual rate in the column of each of Examples 5, 20, and 8, a percentage (%) when the residual rate in the column of each of corresponding Comparative Examples 1, 13, and 4, in which the comparative target capping conditions ([N] × [T] = 5.0) were used, was taken as 100% was also calculated and shown together. When these values are less than 100%, it indicates that the residual rate in the column is suppressed as compared with that of corresponding Comparative Example. As for the purity of the final product of each of Examples 5, 20, and 8, a difference (point) from the purity of the final product of each of Comparative Examples 1, 13, and 4 was also calculated and shown together. When these values are positive values, it indicates that the purity of the final product is improved as compared with that of corresponding Comparative Example.

In Example 5 (charged amount of nucleoside phosphoramidites: 1.2 equivalents), Example 20 (charged amount of nucleoside phosphoramidites: 1.6 equivalents), and Example 8 (charged amount of nucleoside phosphoramidites: 1.7 equivalents), in which the value of [N] × [T] was in the range of 0.5 to 3.0, the percentage of the residual rate in the column relative to Comparative Example was less than 100%, indicating that the residual rate in the column was suppressed, and further, the increase point of the purity of the final product relative to Comparative Example was a positive value, indicating that the purity of the final product was improved. However, these effects were more pronounced when the charged amount of nucleoside phosphoramidites was 1.2 equivalents than when the charged amount was 1.7 equivalents. In particular, as for the effect of improving the purity of the final product as compared with that of Comparative Example, a clear relationship with the charged amount of nucleoside phosphoramidites was observed, and the effect was greater when the charged amount of nucleoside phosphoramidites was 1.6 equivalents (+0.7 points) than when the charged amount was 1.7 equivalents (+0.1 points), and the effect was even greater when the charged amount was 1.2 equivalents (+4.4 points). This phenomenon is discussed below. First, when Comparative Example 1, Comparative Example 13, and Comparative Example 4 were compared, in Comparative Example 4, in which the charged amount of nucleoside phosphoramidites was large, the purity of the final product was as high as 85.8%, and the residual rate in the column was also as high as 33.3%. This indicates that when an excessive amount of nucleoside phosphoramidites are charged, a highly pure final product can be obtained, but it also indicates that a large amount of nucleoside phosphoramidites are not utilized in the synthesis of the final product and remain in the column. On the other hand, Comparative Example 13 and Comparative Example 1 are cases where the charged amount of nucleoside phosphoramidites was reduced relative to Comparative Example 4, and this method focusing only on the economic aspect is not preferable because the purity of the final product decreases to 84.8% or 80.2%. From the above results, it can be understood that in order to economically obtain a highly pure final product, it is required to effectively utilize the charged nucleoside phosphoramidites, that is, to suppress the residual rate in the column. Therefore, the respective Examples are cases where the invention was applied, and as the effect of the invention, the residual rate in the column was suppressed in all of Example 5, Example 20, and Example 8, and the contribution to the increase in purity of the final product was remarkable when the charged amount of nucleoside phosphoramidites was small (an increase in purity was observed when the charged amount was 1.2 equivalents to 1.7 equivalents, and among these, the effect of increasing the purity was greater when the charged amount was 1.2 equivalents to 1.6 equivalents). Although Example 20 (charged amount of nucleoside phosphoramidites: 1.6 equivalents) and Example 5 (charged amount of nucleoside phosphoramidites: 1.2 equivalents) are cases where the charged amount of nucleoside phosphoramidites was reduced to less than 1.7 equivalents, the purity of the final product was increased to 85.5% (Example 20) or 84.6% (Example 5) due to the effect of the invention. It can be said that according to the invention, the residual rate in the column was suppressed and the charged nucleoside phosphoramidites were effectively utilized in the synthesis of the final product, and thus, both quality and economic aspects could be achieved simultaneously.

### 3. Evaluation of Supply Liquid Temperature and Charged Amount Ratio of (Activating Agent/Nucleoside Phosphoramidites)

**[Table 4]**

| Oligonucleotide: SEQ ID NO: 1 | | | | | | | | | | | | | |
|---|---|---|---|---|---|---|---|---|---|---|---|---|---|
| | Supply liquid temperatu re during whole process (°C) | Charged amount of amidites (equivalen ts) | Activating agent | | Charged amount ratio of (activating agent/amidit es) (times) | Blendi ng ratio of NMI in cappin 9 solutio n [N] (%) | Cappi ng time [T] (min) | [N ] × [T] | Evaluation results | | | | |
| | | | Typ e | Charged amount (equivalen ts) | | | | | Residual rate in column | | Purity of final product | | Comparati ve target |
| | | | | | | | | | Residu al rate (%) | Percentag e relative to comparati ve example (%) | Purit y (%) | Increase point relative to comparati ve example | |
| Comparati ve Example 1 | 22.5 | 1.2 | DCI | 4.2 | 3.5 | 10.0 | 0.50 | 5. 0 | 19.9 | 100 | 80.2 | 0 | - |
| Example 5 | 22.5 | 1.2 | DCI | 4.2 | 3.5 | 3.3 | 0.38 | 1. 3 | 14.3 | 72 | 84.6 | 4.4 | relative to Comparati ve Example 1 |
| Example 21 | 22.5 | 1.2 | DCI | 16.8 | 14 | 3.3 | 0.38 | 1. 3 | 12.8 | - | 85.7 | - | - |
| Comparati ve Example 5 | 15.0 | 1.2 | DCI | 16.8 | 14 | 10.0 | 0.50 | 5. 0 | 16.7 | 100 | 87.2 | 0 | - |
| Example 9 | 15.0 | 1.2 | DCI | 16.8 | 14 | 3.3 | 0.38 | 1. 3 | 13.8 | 83 | 90.4 | 3.2 | relative to Comparati ve Example 5 |
| Comparati ve Example 4 | 22.5 | 1.7 | DCI | 6.0 | 3.5 | 10.0 | 0.50 | 5. 0 | 33.3 | 100 | 85.8 | 0 | - |
| Example 8 | 22.5 | 1.7 | DCI | 6.0 | 3.5 | 3.3 | 0.38 | 1. 3 | 25.6 | 77 | 85.9 | 0.1 | relative to Comparati ve Example 4 |
| Example 22 | 22.5 | 1.7 | DCI | 23.8 | 14 | 3.3 | 0.38 | 1. 3 | 17.9 | - | 87.1 | - | - |
| Comparati ve Example 6 | 15.0 | 1.7 | DCI | 23.8 | 14 | 10.0 | 0.50 | 5. 0 | 24.8 | 100 | 91.2 | 0 | - |
| Example 10 | 15.0 | 1.7 | DCI | 23.8 | 14 | 3.3 | 0.38 | 1. 3 | 21.1 | 85 | 92.5 | 1.3 | relative to Comparati ve Example 6 |

| | | | | | | | | | | | | | |
|---|---|---|---|---|---|---|---|---|---|---|---|---|---|
| Activating agent: DCI (4,5-dicyanoimidazole), Amidite: nucleoside phosphoramidite Blending ratio of capping solution component (base): 15% lutidine, Blending ratio of capping solution component (acetylating agent): 10% acetic anhydride NMI: N-methylimidazole | | | | | | | | | | | | | |

As the evaluation results of Examples 5, 8, 9, 10, 21, and 22 and Comparative Examples 1, 4, 5, and 6, the residual rate (%) in the column and the purity (%) of the final product are shown in Table 4. As for the residual rate in the column of each of Examples 5, 8, 9, and 10, a percentage (%) when the residual rate in the column of each of corresponding Comparative Examples 1, 4, 5, and 6, in which the comparative target capping conditions ([N] × [T] = 5.0) were used, was taken as 100% was also calculated and shown together. When these values are less than 100%, it indicates that the residual rate in the column is suppressed as compared with that of corresponding Comparative Example. As for the purity of the final product of each of Examples 5, 8, 9, and 10, a difference (point) from the purity of the final product of each of Comparative Examples 1, 4, 5, and 6 was also calculated and shown together. When these values are positive values, it indicates that the purity of the final product is improved as compared with that of corresponding Comparative Example.

In Example 5 (charged amount of nucleoside phosphoramidites: 1.2 equivalents, supply liquid temperature during whole process: 22.5°C, charged amount ratio of (activating agent/nucleoside phosphoramidites): 3.5 times), Example 8 (charged amount of nucleoside phosphoramidites: 1.7 equivalents, supply liquid temperature during whole process: 22.5°C, charged amount ratio of (activating agent/nucleoside phosphoramidites): 3.5 times), Example 9 (charged amount of nucleoside phosphoramidites: 1.2 equivalents, supply liquid temperature during whole process: 15°C, charged amount ratio of (activating agent/nucleoside phosphoramidites): 14 times), and Example 10 (charged amount of nucleoside phosphoramidites: 1.7 equivalents, supply liquid temperature during whole process: 15°C, charged amount ratio of (activating agent/nucleoside phosphoramidites): 14 times), in which the value of [N] × [T] was in the range of 0.5 to 3.0, the percentage of the residual rate in the column relative to Comparative Example was less than 100%, indicating that the residual rate in the column was suppressed, and further, the increase point of the purity of the final product relative to Comparative Example was a positive value, indicating that the purity of the final product was improved. Further, in Example 5 (charged amount of nucleoside phosphoramidites: 1.2 equivalents, supply liquid temperature during whole process: 22.5°C, charged amount ratio of (activating agent/nucleoside phosphoramidites): 3.5 times), the residual rate in the column was 14.3% and the purity of the final product was 84.6%, whereas in Example 21 (charged amount of nucleoside phosphoramidites: 1.2 equivalents, supply liquid temperature during whole process: 22.5°C, charged amount ratio of (activating agent/nucleoside phosphoramidites): 14 times) and Example 9 (charged amount of nucleoside phosphoramidites: 1.2 equivalents, supply liquid temperature during whole process: 15°C, charged amount ratio of (activating agent/nucleoside phosphoramidites): 14 times), the residual rate in the column was further suppressed to 12.8% (Example 21) or 13.8% (Example 9), and the purity of the final product was improved to 85.7% (Example 21) or 90.4% (Example 9). Therefore, it was found that by lowering the supply liquid temperature and/or increasing the charged amount ratio of (activating agent/nucleoside phosphoramidites), the residual rate in the column can be further suppressed and the purity of the final product can be further improved as compared with the case of relaxing the capping conditions alone. In Example 8 (charged amount of nucleoside phosphoramidites: 1.7 equivalents, supply liquid temperature during whole process: 22.5°C, charged amount ratio of (activating agent/nucleoside phosphoramidites): 3.5 times), the residual rate in the column was 25.6% and the purity of the final product was 85.9%, whereas in Example 22 (charged amount of nucleoside phosphoramidites: 1.7 equivalents, supply liquid temperature during whole process: 22.5°C, charged amount ratio of (activating agent/nucleoside phosphoramidites): 14 times) and Example 10 (charged amount of nucleoside phosphoramidites: 1.7 equivalents, supply liquid temperature during whole process: 15°C, charged amount ratio of (activating agent/nucleoside phosphoramidites): 14 times), the residual rate in the column was suppressed to 17.9% (Example 22) or 21.1% (Example 10), and the purity of the final product was increased to 87.1% (Example 22) or 92.5% (Example 10) in the same manner. Therefore, it was found that by lowering the supply liquid temperature and/or increasing the charged amount ratio of (activating agent/nucleoside phosphoramidites), the residual rate in the column can be further suppressed and the purity of the final product can be improved as compared with the case of relaxing the capping conditions alone.

From the above results, it was found that in the production of an oligonucleotide, the effect of suppressing the residual rate of nucleoside phosphoramidites in a column and further the effect capable of improving the purity of the final synthesized oligonucleotide product by performing capping under relaxed capping conditions in which the value of [N] × [T] is 0.5 to 3.0 can be further enhanced by lowering the supply liquid temperature and/or increasing the charged amount ratio of (activating agent/nucleoside phosphoramidites).

### 4. Evaluation of Effect of Capping Solution Components Other than NMI

**[Table 5]**

| | Blending ratio of capping solution components | | | | Cappin g time [T] (min) | [N ] × [T] | Evaluation results | | | | |
|---|---|---|---|---|---|---|---|---|---|---|---|
| | Blendin g ratio of NMI [N] (%) | Base | | Blendin g ratio of acetic anhydri de (%) | | | Residual rate in column | | Purity of final product | | Comparati ve target |
| | | Type | Blendin g ratio (%) | | | | Residu al rate (%) | Percentag e relative to comparati ve example (%) | Purit y (%) | Increase point relative to comparati ve example | |
| Comparati ve Example 1 | 10.0 | Lutidin e | 15.0 | 10.0 | 0.50 | 5. 0 | 19.9 | 100 | 80.2 | 0 | - |
| Example 5 | 3.3 | Lutidin e | 15.0 | 10.0 | 0.38 | 1. 3 | 14.3 | 72 | 84.6 | 4.4 | relative to Comparati ve Example 1 |
| Example 11 | 3.3 | Lutidin e | 5.0 | 10.0 | 0.38 | 1. 3 | 13.2 | 66 | 84.2 | 4.0 | relative to Comparati ve Example 1 |
| Example 12 | 3.3 | Lutidin e | 22.5 | 10.0 | 0.38 | 1. 3 | 14.8 | 74 | 84.3 | 4.1 | relative to Comparati ve Example 1 |
| Example 13 | 3.3 | Pyridin e | 15.0 | 10.0 | 0.38 | 1. 3 | 12.6 | 63 | 85.7 | 5.5 | relative to Comparati ve Example 1 |
| Comparati ve Example 14 | 10.0 | Lutidin e | 15.0 | 5.0 | 0.50 | 5. 0 | 15.7 | 100 | 79.6 | 0.0 | |
| Example 23 | 3.3 | Lutidin e | 15.0 | 5.0 | 0.38 | 1. 3 | 11.8 | 75 | 82.2 | 2.6 | relative to Comparati ve Example 14 |
| Comparati ve Example 15 | 10.0 | Lutidin e | 15.0 | 2.5 | 0.50 | 5. 0 | 13.3 | 100 | 80.4 | 0.0 | |
| Example 24 | 3.3 | Lutidin e | 15.0 | 2.5 | 0.38 | 1. 3 | 11.7 | 88 | 82.9 | 2.5 | relative to Comparati ve Example 15 |

| | | | | | | | | | | | |
|---|---|---|---|---|---|---|---|---|---|---|---|
| Oligonucleotide: SEQ ID NO: 1, Supply liquid temperature during whole process: 22.5°C, Charged amount of nucleoside phosphoramidites: 1.2 equivalents, Activating agent: DCI (4,5-dicyanoimidazole): 4.2 equivalents, Charged amount ratio of (activating agent/nucleoside phosphoramidites): 3.5 times, NMI: N-methylimidazole | | | | | | | | | | | |

As the evaluation results of Examples 5, 11 to 13, 23, and 24 and Comparative Examples 1, 14, and 15, the residual rate (%) in the column and the purity (%) of the final product are shown in Table 5. As for the residual rate in the column of each of Examples 5, and 11 to 13, a percentage (%) when the residual rate in the column of Comparative Example 1, in which the blending ratio of acetic anhydride was 10% and the comparative target capping conditions ([N] × [T] = 5.0) were used, was taken as 100% was also calculated and shown together. As for the residual rate in the column of each of Examples 23 and 24, a percentage (%) when the residual rate in the column of Comparative Example 14 or 15, in which the blending ratio of acetic anhydride was 5% or 2.5% and the comparative target capping conditions ([N] × [T] = 5.0) were used, was taken as 100% was also calculated and shown together. When these values are less than 100%, it indicates that the residual rate in the column is suppressed as compared with that of Comparative Example. As for the purity of the final product of each of Examples 5, and 11 to 13, a difference (point) from the purity of the final product of Comparative Example 1 was also calculated and shown together. As for the purity of the final product of each of Examples 23 and 24, a difference (point) from the purity of the final product of Comparative Example 14 or 15 was also calculated and shown together. When these values are positive values, it indicates that the purity of the final product is improved as compared with that of corresponding Comparative Example.

In each of Example 5 (containing 15% lutidine), Example 11 (containing 5% lutidine), Example 12 (containing 22.5% lutidine), and Example 13 (containing 15% pyridine), in which the blending ratio of acetic anhydride was 10% and the value of [N] × [T] was in the range of 0.5 to 3.0, the percentage of the residual rate in the column relative to Comparative Example (Comparative Example 1 in which the blending ratio of acetic anhydride was 10%) was less than 100%, indicating that the residual rate in the column was suppressed as the effect of the invention, and further, the increase point of the purity of the final product relative to Comparative Example (Comparative Example 1) was a positive value, indicating that the purity of the final product was improved as the effect of the invention. In each of Example 23 (containing 5% acetic anhydride and 15% lutidine) and Example 24 (containing 2.5% acetic anhydride and 15% lutidine), in which the blending ratio of acetic anhydride was reduced to 5% or 2.5%, and the value of [N] × [T] was in the range of 0.5 to 3.0, the percentage of the residual rate in the column relative to Comparative Example (Comparative Example 14 in which the blending ratio of acetic anhydride was 5%, or Comparative Example 15 in which the blending ratio of acetic anhydride was 2.5%) was less than 100%, indicating that the residual rate in the column was suppressed as the effect of the invention, and further, the increase point of the purity of the final product relative to Comparative Example (Comparative Example 14 or 15) was a positive value, indicating that the purity of the final product was improved as the effect of the invention. These effects did not differ significantly among Examples 5, 11 to 13, 23, and 24, in which the formulations of the capping solution components other than N-methylimidazole were varied. Comparative Example 14 (blending ratio of acetic anhydride: 5%) and Comparative Example 15 (blending ratio of acetic anhydride: 2.5%) are cases where the blending ratio of acetic anhydride was reduced from that of Comparative Example 1 (blending ratio of acetic anhydride: 10%), and while the purity of the final product of Comparative Example 1 was 80.2%, the purities of the final product of Comparative Example 14 and Comparative Example 15 were 79.6% and 80.4%, respectively, and the purity of the final product was not improved by reducing the blending ratio of acetic anhydride. That is, it could be verified that the invention of this application, in which the value of [N] × [T] is controlled within the range of 0.5 to 3.0 rather than reducing the blending ratio of acetic anhydride, is useful for improving the purity of the final product.

From the above results, it was found that in the production of an oligonucleotide, the effect of suppressing the residual rate of nucleoside phosphoramidites in a column and further the effect capable of improving the purity of the final synthesized oligonucleotide product by performing capping under relaxed capping conditions in which the value of [N] × [T] is 0.5 to 3.0 were hardly affected by the capping solution components other than N-methylimidazole.

### 5. Evaluation of Effect of Sulfurization of Oligonucleotide

**[Table 6]**

| | Oligonucl eotide | Charged amount of amidites (equival ents) | Charged amount of activatin g agent (equival ents) | Charged amount ratio of (activating agent/ami dites) (times) | Blend ing ratio of NMI in cappi ng soluti on [N] (%) | Capp ing time [T] (min) | [ N × [T ] | Evaluation results | | | | |
|---|---|---|---|---|---|---|---|---|---|---|---|---|
| | | | | | | | | Residual rate in column | | Purity of final product | | Compar ative target |
| | | | | | | | | Resid ual rate (%) | Percent age relative to compar ative exampl e (%) | Pur ity (%) | Increas e point relative to compar ative exampl e | |
| Compar ative Exampl e 1 | SEQ ID NO: 1 | 1.2 | 4.2 | 3.5 | 10.0 | 0.50 | 5. 0 | 19.9 | 100 | 80. 2 | 0 | - |
| Exampl e 5 | SEQ ID NO: 1 | 1.2 | 4.2 | 3.5 | 3.3 | 0.38 | 1. 3 | 14.3 | 72 | 84. 6 | 4.4 | relative to Compar ative Exampl e 1 |
| Compar ative Exampl e 4 | SEQ ID NO: 1 | 1.7 | 6.0 | 3.5 | 10.0 | 0.50 | 5. 0 | 33.3 | 100 | 85. 8 | 0 | - |
| Exampl e 8 | SEQ ID NO: 1 | 1.7 | 6.0 | 3.5 | 3.3 | 0.38 | 1. 3 | 25.6 | 77 | 85. 9 | 0.1 | relative to Compar ative Exampl e 4 |
| Compar ative Exampl e 7 | SEQ ID NO: 2 | 1.2 | 4.2 | 3.5 | 10.0 | 0.50 | 5. 0 | 8.9 | 100 | 77. 9 | 0 | - |
| Exampl e 14 | SEQ ID NO: 2 | 1.2 | 4.2 | 3.5 | 3.3 | 0.38 | 1. 3 | 0.3 | 3 | 75. 1 | -2.8 | relative to Compar ative Exampl e 7 |
| Compar ative Exampl e 8 | SEQ ID NO: 2 | 1.7 | 6.0 | 3.5 | 10.0 | 0.50 | 5. 0 | 20.0 | 100 | 81. 1 | 0 | - |
| Exampl e 15 | SEQ ID NO: 2 | 1.7 | 6.0 | 3.5 | 3.3 | 0.38 | 1. 3 | 11.0 | 55 | 80. 1 | -1.0 | relative to Compar ative Exampl e 8 |
| Compar ative Exampl e 16 | SEQ ID NO: 7 | 1.2 | 4.2 | 3.5 | 10.0 | 0.50 | 5. 0 | 8.4 | 100 | 76. 9 | 0.0 | |
| Exampl e 25 | SEQ ID NO: 7 | 1.2 | 4.2 | 3.5 | 3.3 | 0.38 | 1. 3 | 6.8 | 81 | 79. 7 | 2.8 | relative to Compar ative Example 16 |
| Compar ative Exampl e 17 | SEQ ID NO: 8 | 1.2 | 4.2 | 3.5 | 10.0 | 0.50 | 5. 0 | 12.8 | 100 | 82. 1 | 0.0 | |
| Exampl e 26 | SEQ ID NO: 8 | 1.2 | 4.2 | 3.5 | 3.3 | 0.38 | 1. 3 | 6.3 | 49 | 83. 2 | 1.1 | relative to Compar ative Exampl e 17 |

| | | | | | | | | | | | | |
|---|---|---|---|---|---|---|---|---|---|---|---|---|
| Supply liquid temperature during whole process: 22.5°C, Activating agent: DCI (4,5-dicyanoimidazole), Amidite: nucleoside phosphoramidite Blending ratio of capping solution component (base): 15% lutidine, Blending ratio of capping solution component (acetylating agent): 10% acetic anhydride NMI: N-methylimidazole | | | | | | | | | | | | |

As the evaluation results of Examples 5, 8, 14, 15, 25, and 26 and Comparative Examples 1, 4, 7, 8, 16, and 17, the residual rate (%) in the column and the purity (%) of the final product are shown in Table 6. As for the residual rate in the column of each of Examples 5, 8, 14, 15, 25, and 26, a percentage (%) when the residual rate in the column of each of corresponding Comparative Examples 1, 4, 7, 8, 16, and 17, in which the comparative target capping conditions ([N] × [T] = 5.0) were used, was taken as 100% was also calculated and shown together. When these values are less than 100%, it indicates that the residual rate in the column is suppressed as compared with that of corresponding Comparative Example. As for the purity of the final product of each of Examples 5, 8, 14, 15, 25, and 26, a difference (point) from the purity of the final product of each of Comparative Examples 1, 4, 7, 8, 16, and 17 was also calculated and shown together. When these values are positive values, it indicates that the purity of the final product is improved as compared with that of corresponding Comparative Example.

In Example 5 (charged amount of nucleoside phosphoramidites: 1.2 equivalents, SEQ ID NO: 1 (with sulfurization of all linkage portions P), Example 8 (charged amount of nucleoside phosphoramidites: 1.7 equivalents, SEQ ID NO: 1 (with sulfurization of all linkage portions P), Example 14 (charged amount of nucleoside phosphoramidites: 1.2 equivalents, SEQ ID NO: 2 (without sulfurization of any of linkage portions P), Example 15 (charged amount of nucleoside phosphoramidites: 1.7 equivalents, SEQ ID NO: 2 (without sulfurization of any of linkage portions P), Example 25 (charged amount of nucleoside phosphoramidites: 1.2 equivalents, SEQ ID NO: 7 (with sulfurization of linkage portions P at 17 sites of terminal portions out of all linkage portions P at 23 sites ((17 sites/23 sites) × 100 = 74%))), and Example 26 (charged amount of nucleoside phosphoramidites: 1.2 equivalents, SEQ ID NO: 8 (with sulfurization of linkage portions P at 18 sites of central portions out of all linkage portions P at 23 sites ((18 sites/23 sites) × 100 = 78%))), in which the value of [N] × [T] was in the range of 0.5 to 3.0, the percentage of the residual rate in the column relative to Comparative Example was less than 100%, indicating that the residual rate in the column was suppressed. However, in Example 5, Example 8, Example 25, and Example 26, in which conditions with sulfurization were used, the increase point of the purity of the final product relative to Comparative Example was a positive value, and the purity of the final product was improved, whereas in Example 14 and Example 15, in which conditions without sulfurization were used, the increase point was not a positive value, and the purity of the final product was not improved.

From the above results, it was found that in the production of an oligonucleotide, by performing capping under relaxed capping conditions in which the value of [N] × [T] is 0.5 to 3.0, the residual rate of nucleoside phosphoramidites in the column can be suppressed. It was further found that by sulfurization of an oligonucleotide, the purity of the final synthesized oligonucleotide product can be improved. In particular, it was found that the purity of the final synthesized oligonucleotide product can be improved by sulfurization of linkage portions P at 74% or more of the sites of an oligonucleotide, by sulfurization of linkage portions P at 78% or more of the sites, by sulfurization of linkage portions P at 17 or more sites, or by sulfurization of linkage portions P at 18 or more sites.

### 6. Evaluation of Sequence of Oligonucleotide

**[Table 7]**

| | Oligonucleoti de | Activating agent | | Charged amount ratio of (activating agent/amidite s) (times) | Blendin g ratio of NMI in capping solution [N] (%) | Cappin g time [T] (min) | [N ] × [T] | Evaluation results | | | | |
|---|---|---|---|---|---|---|---|---|---|---|---|---|
| | | Typ e | Charged amount (equivalent s) | | | | | Residual rate in column | | Purity of final product | | Comparativ e target |
| | | | | | | | | Residu al rate (%) | Percentag e relative to comparativ e example (%) | Purit y (%) | Increase point relative to comparativ e example | |
| Comparativ e Example 1 | SEQ ID NO: 1 | DCI | 4.2 | 3.5 | 10.0 | 0.50 | 5. 0 | 19.9 | 100 | 80.2 | 0 | - |
| Example 5 | SEQ ID NO: 1 | DCI | 4.2 | 3.5 | 3.3 | 0.38 | 1. 3 | 14.3 | 72 | 84.6 | 4.4 | relative to Comparativ e Example 1 |
| Comparativ e Example 9 | SEQ ID NO: 3 | DCI | 4.2 | 3.5 | 10.0 | 0.50 | 5. 0 | 13.3 | 100 | 80.8 | 0 | - |
| Example 16 | SEQ ID NO: 3 | DCI | 4.2 | 3.5 | 3.3 | 0.38 | 1. 3 | 8.1 | 61 | 82.9 | 2.1 | relative to Comparativ e Example 9 |
| Comparativ e Example 10 | SEQ ID NO: 4 | DCI | 4.2 | 3.5 | 10.0 | 0.50 | 5. 0 | 18.3 | 100 | 84.6 | 0 | - |
| Example 17 | SEQ ID NO: 4 | DCI | 4.2 | 3.5 | 3.3 | 0.38 | 1. 3 | 13.7 | 75 | 85.3 | 0.7 | relative to Comparativ e Example 10 |
| Comparativ e Example 11 | SEQ ID NO: 5 | DCI | 4.2 | 3.5 | 10.0 | 0.50 | 5. 0 | 17.5 | 100 | 79.8 | 0 | - |
| Example 18 | SEQ ID NO: 5 | DCI | 4.2 | 3.5 | 3.3 | 0.38 | 1. 3 | 10.0 | 57 | 80.5 | 0.7 | relative to Comparativ e Example 11 |
| Comparativ e Example 12 | SEQ ID NO: 6 | ETT | 3.6 | 3.0 | 10.0 | 0.50 | 5. 0 | 20.4 | 100 | 93.2 | 0 | - |
| Example 19 | SEQ ID NO: 6 | ETT | 3.6 | 3.0 | 3.3 | 0.38 | 1. 3 | 10.4 | 51 | 94.3 | 1.1 | relative to Comparativ e Example 12 |

| | | | | | | | | | | | | |
|---|---|---|---|---|---|---|---|---|---|---|---|---|
| Supply liquid temperature during whole process: 22.5°C, Charged amount of amidites: 1.2 equivalents, Amidite: nucleoside phosphoramidite DCI (4,5-dicyanoimidazole), ETT: 5-(ethylthio)-1H-tetrazole Blending ratio of capping solution component (base): 15% lutidine, Blending ratio of capping solution component (acetylating agent): 10% acetic anhydride NMI: N-methylimidazole | | | | | | | | | | | | |

As the evaluation results of Examples 5, and 16 to 19 and Comparative Examples 1, and 9 to 12, the residual rate (%) in the column and the purity (%) of the final product are shown in Table 7. As for the residual rate in the column of each of Examples 5, and 16 to 19, a percentage (%) when the residual rate in the column of each of corresponding Comparative Examples 1, and 9 to 12, in which the comparative target capping conditions ([N] × [T] = 5.0) were used, was taken as 100% was also calculated and shown together. When these values are less than 100%, it indicates that the residual rate in the column is suppressed as compared with that of corresponding Comparative Example. As for the purity of the final product of each of Examples 5, and 16 to 19, a difference (point) from the purity of the final product of each of Comparative Examples 1, and 9 to 12 was also calculated and shown together. When these values are positive values, it indicates that the purity of the final product is improved as compared with that of corresponding Comparative Example.

In each of Example 5 (SEQ ID NO: 1, activating agent: DCI), Example 16 (SEQ ID NO: 3, activating agent: DCI), Example 17 (SEQ ID NO: 4, activating agent: DCI), Example 18 (SEQ ID NO: 5, activating agent: DCI), and Example 19 (SEQ ID NO: 6, activating agent: ETT), in which the value of [N] × [T] was in the range of 0.5 to 3.0, the percentage of the residual rate in the column relative to Comparative Example was less than 100%, indicating that the residual rate in the column was suppressed, and further, the increase point of the purity of the final product relative to Comparative Example was a positive value, indicating that the purity of the final product was improved. These effects did not differ significantly among Examples 5, and 16 to 19, in which the nucleic acid type, sequence, chain length, presence or absence of modification, and activating agent type for the oligonucleotide were varied.

From the above results, it was found that in the production of an oligonucleotide, the effect of suppressing the residual rate of nucleoside phosphoramidites in a column and further the effect capable of improving the purity of the final synthesized oligonucleotide product by performing capping under relaxed capping conditions in which the value of [N] × [T] is 0.5 to 3.0 were hardly affected by the nucleic acid type, sequence, chain length, presence or absence of modification, and activating agent type for the oligonucleotide.

## Claims

1. A method for producing an oligonucleotide, the method comprising:
(a) a step of detaching a protecting group from a protected nucleoside in which the protecting group is bonded to a hydroxy group, a thiol group, or an amino group at a 3'-position or a 5'-position;
(b) a step of bonding a nucleoside phosphoramidite to the hydroxy group, the thiol group, or the amino group at the 3'-position or the 5'-position of the nucleoside, from which the protecting group has been detached, in the presence of an activating agent;
(c) a step of sulfurizing or oxidizing a bond formed in the step (b); and
(d) a step of capping an unbonded hydroxy group, thiol group, or amino group at the 3'-position or the 5'-position of the nucleoside, wherein
the capping in the step (d) includes an operation of bringing the nucleoside and a capping solution into contact with each other for only a predetermined time [T] (minutes),
the capping solution contains N-methylimidazole at a blending ratio [N] (%) (which is a percentage (%) of a room temperature volume of N-methylimidazole before blending relative to a sum (100%) of room temperature volumes before blending of respective reagents to be blended as capping solution components), and
a product of [N] and [T] ([N] × [T]) is 0.5 to 3.0.

2. The method according to claim 1, wherein the oligonucleotide has a sulfurized internucleoside bond.

3. The method according to claim 1 or 2, wherein [N] is 1.1 to 8.0 (%).

4. The method according to any one of claims 1 to 3, wherein [T] is 0.1 to 0.5 (minutes).

5. The method according to any one of claims 1 to 4, wherein the temperature of the solution in any of the steps (a) to (d) is 0 to 20°C.

6. The method according to any one of claims 1 to 5, wherein the amount of the activating agent used in the step (b) is 10.0 to 15.0 equivalents of the amount of nucleoside phosphoramidites used in the step (b).

7. The method according to any one of claims 1 to 6, wherein the amount of the activating agent used in the step (b) is 10.0 to 25.0 equivalents of the supported nucleoside in the step (b).

8. The method according to any one of claims 1 to 7, wherein the activating agent in the step (b) is selected from a group consisting of 4,5-dicyanoimidazole and 5-(ethylthio)-1H-tetrazole.
